# EUROPEAN PATENT APPLICATION

(11) **EP 2 439 194 A1**
(43) Date of publication of application: **11.04.2012**
(21) Application number: 10186578.0
(22) Date of filing: 05.10.2010
(51) Int. Cl.: C07D 213/81, C07D 307/68, C07C 233/80, C07D 307/54, C07D 317/60, C07D 319/18, A61K 31/4409, A61K 31/341, A61K 31/357, A61P 35/00

(54) **Phenyl diamides and a pharmaceutical preparation comprising phenyl diamides**

(71) Applicant: Rheinische Friedrich-Wilhelms Universität, 53113 Bonn (DE)
(72) Inventor: Famulok, Michael, 53175, Bonn (DE); Bill, Anke, 53121, Bonn (DE); Schmitz, Anton, 53121, Bonn (DE); Bajorath, Jürgen, 53111 Bonn (DE)
(74) Representative: Michalski Hüttermann & Partner

(57) **Abstract**

The present invention relates to phenyl diamides according to the general formula (1) and their use as a medicament and a pharmaceutical preparation comprising as an active ingredient a phenyl diamide according to the general formula (1). Further, the present invention relates to phenyl diamides according to the formulas (16), (17) and (18) for use in the treatment of gliomas.

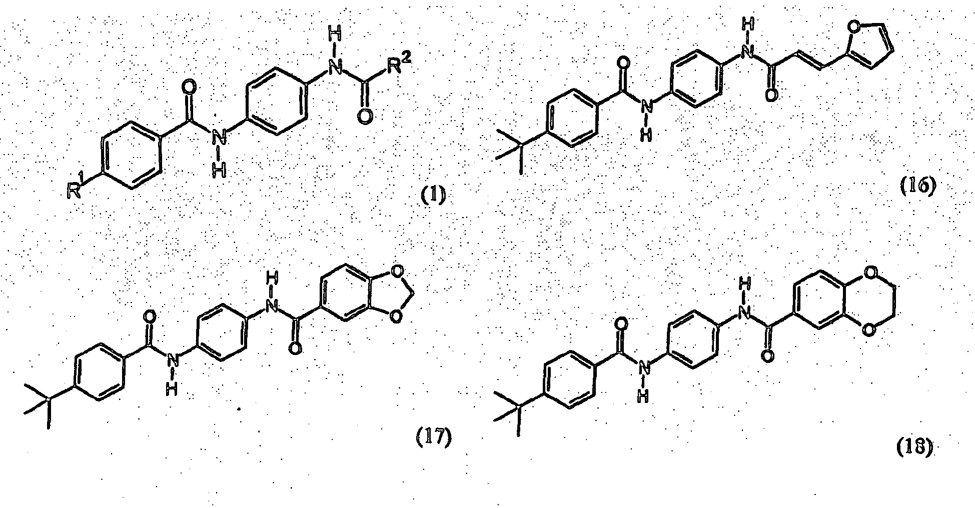

## Description

The present invention relates to phenyl diamides and a pharmaceutical preparation comprising as an active ingredient such phenyl diamides. In particular, the present invention relates to the use of phenyl diamides for the treatment of tumor diseases, especially gliomas.

Gliomas are the most common subtype of primary brain tumors and are responsible for about 2 % of all deaths from cancer. Gliomas are classified according to their line of differentiation and are afterward graded according to their malignancy. Glioblastomas are the most frequent form of gliomas and represent grade IV astrocytomas. As all grade IV tumors glioblastomas are highly malignant, usually resistant to chemotherapy and infiltrate throughout the brain. This invasive nature results in the inability of surgery and the very short median survival of 9 to 12 months of glioblastoma patients.

The epidermal growth factor receptor (EGFR) is a primary contributor to glioblastoma initiation and progression. EGFR amplification and overexpression is the most common genetic alteration in primary glioblastoma with a frequency of 40 % to 70 %, but is not observed in lower grade astrocytomas. Epidermal growth factor receptors are key regulators of cell differentiation, proliferation and migration. The epidermal growth factor receptor is a transmembrane glycoprotein whose activation or autophosphorylation triggers a signalling cascade correlating with increased proliferation of tumour cells. Epidermal growth factor receptor inhibitors therefore have become an important class of drugs used in cancer therapy.

The invasive nature and high malignancy of glioblastomas together with the resistance to classical chemo- and radiotherapy contribute to the very poor prognosis of glioblastoma patients. However, a high prevalence of the most abundant mutation EGFR variant III (EGFRvIII), which is found in approximately 50 % to 60 % of glioblastoma that overexpress the EGFR but not in normal tissue, make the epidermal growth factor receptor a very attractive therapeutic target. For example, MAb806, aEGFRvIII-targeting monoclonal antibody, led to tumor shrinkage in subcutaneous and orthotopic tumor models and is currently under investigation for clinical use. Further, several EGFR specific tyrosine kinase inhibitors have also been evaluated for their efficacy against gliomas but they appear relatively ineffective for tumours expressing EGFRvIII.

Therefore, the objective underlying the present invention was to provide novel compounds being usable in a pharmacological treatment of gliomas.

The problem is solved by phenyl diamides according to the general formula (1) as indicated below and/or racemates, enantiomers, diastereomers, solvates, hydrates, and pharmaceutically acceptable salts and/or esters thereof: 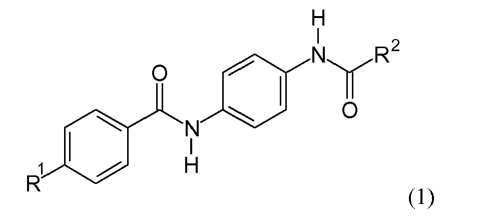 wherein:
- R¹: is selected from the group comprising hydrogen, linear or branched C₁-C₁₀-alkyl and/or linear or branched C₂-C₁₀-alkenyl;
- R²: is selected from the group comprising structural elements (2), (3) and/or (4) as given as follows: 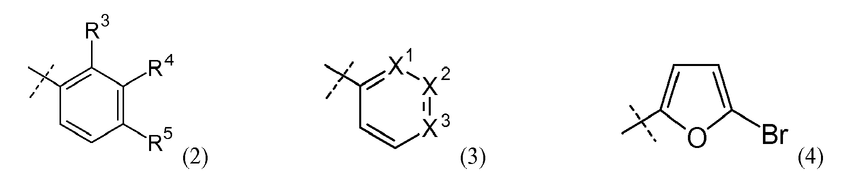 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH,
for use as a medicament.

It was surprisingly found that the phenyl diamides according to the general formula (1) exhibit a strong inhibition of the guanine nucleotide exchange factors of the family of cytohesins and are potent inhibitors of glioblastoma cell proliferation.

The present invention therefore provides a novel approach to the treatment of cancer, especially gliomas.

The term "alkyl" according to the invention is to be understood as meaning straight-chain or branched alkyl groups. The term "C₁-C₁₀-alkyl" as used herein refers to straight-chain or branched alkyl groups having 1 to 10 carbon atoms. Preferred C₁-C₁₀-alkyl groups are selected from the group comprising methyl, ethyl and the isomers of propyl, butyl, pentyl, hexyl, heptyl or octyl, such as, for example, isopropyl, isobutyl, tert.-butyl, sec.-butyl and/or isopentyl. Especially preferred are C₁-C₅-alkyl groups selected from the group comprising methyl, ethyl, isopropyl and/or tert.-butyl.

The term "alkenyl" according to the invention is to be understood as meaning straight-chain or branched alkyl groups having at least one or several double bonds. The term "C₂-C₁₀₋alkenyl" as used herein refers to straight-chain or branched alkenyl groups having 2 to 10 carbon atoms and at least one or several double bonds. A preferred C₂-C₁₀-alkenyl is propen-1-yl.

The term "halogen" according to the invention is to be understood as meaning fluorine, chlorine, bromine or iodine, preferably fluorine, chlorine or bromine.

Preferably, the substituent R¹ is a straight-chain or branched C₁-C₅-alkyl group selected from the group comprising methyl, ethyl, isopropyl and/or tert.-butyl, preferably a branched C₁-C₅-alkyl group selected from the group comprising isopropyl and/or tert.-butyl. Most preferably, substituent R¹ is tert.-butyl.

Advantageously, the phenyl diamides according to the general formula (1) wherein R¹ is tert.-butyl showed a strong inhibition of the guanine nucleotide exchange factors of the family of cytohesins and showed to be strong inhibitors of glioblastoma cell proliferation.

Without being bound to a special theory, it is assumed that it is essential for the effect on the proliferation of glioblastoma cells that the phenyl diamides according to the general formula (1) inhibit a class of proteins called cytohesins. Cytohesins are supposed to have a regulatory function on the family of ErbB receptors. Cytohesins have been found to be cytoplasmic ErbB-receptor activators and an inhibition of these proteins results a decreased receptor activation and signalling. Particularly, it was found that cytohesin inhibitors inhibit the autophosphorylation and function of the epidermal growth factor receptor. As epidermal growth factor receptor activation or autophosphorylation correlates with increased proliferation of tumour cells epidermal growth factor receptor inhibitors are potentially usable in cancer therapy.

In a preferred embodiment, the phenyl diamides comprise a substituent R² that is the structural element (2). Advantageously, the phenyl diamides according to the general formula (1) wherein R² is the structural element (2) showed to be potent inhibitors of glioblastoma cell proliferation.

In a further preferred embodiment, the phenyl diamides comprise a substituent R² that is the structural element (2), wherein R³ is selected from the group comprising Cl, F, linear or branched C₁-C₄-alkyl and/or NO₂ and R⁴ and R⁵ are hydrogen.

Advantageously, the phenyl diamides according to the general formula (1) wherein R² is the structural element (2) and wherein R³ is selected from the group comprising Cl, F, linear or branched C₁-C₄-alkyl and/or NO₂ and R⁴ and R⁵ are hydrogen showed to be potent inhibitors of glioblastoma cell proliferation.

In a further preferred embodiment, the phenyl diamides comprise a substituent R² that is the structural element (2), wherein R³ and R⁵ are hydrogen, and R⁴ is selected from the group comprising Cl, F, linear or branched C₁-C₄-alkyl and/or NO₂, preferably Cl.

Advantageously, the phenyl diamides according to the general formula (1) wherein R² is the structural element (2) and wherein R³ and R⁵ are hydrogen, and R⁴ is selected from the group comprising Cl, F, linear or branched C₁-C₄-alkyl and/or NO₂, preferably Cl showed to be potent inhibitors of glioblastoma cell proliferation. Preferably, a proliferation inhibition of > 70 % compared to untreated glioblastoma cells could be achieved.

In a further preferred embodiment, the phenyl diamides comprise a substituent R² that is the structural element (2), wherein R³ and R⁴ are hydrogen, and R⁵ is selected from the group comprising Cl, F, linear or branched C₁-C₄-alkyl and/or NO₂.

Advantageously, the phenyl diamides according to the general formula (1) wherein R² is the structural element (2) and wherein R³ and R⁴ are hydrogen, and R⁵ is selected from the group comprising Cl, F, linear or branched C₁-C₄-alkyl and/or NO₂ showed to be potent inhibitors of glioblastoma cell proliferation.

In a further preferred embodiment, the phenyl diamides comprise a substituent R² that is the structural element (2), wherein R⁴ is hydrogen, and R³ and R⁵ are selected from the group comprising Cl, F, linear or branched C₁-C₄-alkyl and/or NO₂, preferably methyl.

Advantageously, the phenyl diamides according to the general formula (1) wherein R² is the structural element (2) and wherein R⁴ is hydrogen, and R³ and R⁵ are selected from the group comprising Cl, F, linear or branched C₁-C₄-alkyl and/or NO₂, especially methyl showed to be potent inhibitors of glioblastoma cell proliferation. Preferably, an IC₅₀ for the proliferation inhibition of < 2 µM could be achieved.

In other preferred embodiments, the phenyl diamides according to the general formula (1) comprise a substituent R² that is the structural element (3). In a further preferred embodiment, the phenyl diamides comprise a substituent R² that is the structural element (3), wherein X¹ and X² are CH and X³ is N.

It was surprisingly found that the phenyl diamides according to the general formula (1) wherein R² is the structural element (3) and wherein X¹ and X² are CH and X³ is N can exhibit a selectivity for the mutation EGFR variant III (EGFRvIII). Such selectivity for the EGFRvIII mutation can be especially useful in a pharmacological treatment of gliomas.

Alternatively, X¹ and X³ can be CH and X² N, or X² and X³ can be CH and X¹ N. In further embodiment, X¹ and X³, X¹ and X², or X² and X³ can be N.

In a further preferred embodiment, the phenyl diamides comprise a substituent R² that is the structural element (4). Advantageously, the phenyl diamides according to the general formula (1) wherein R² is the structural element (4) can exhibit a selectivity for the mutation EGFR variant III (EGFRvIII).

In more preferred embodiments, the phenyl diamides are selected from the group comprising the compounds (5) to (15) as indicated below and/or racemates, enantiomers, diastereomers, solvates, hydrates, and pharmaceutically acceptable salts and/or esters thereof:

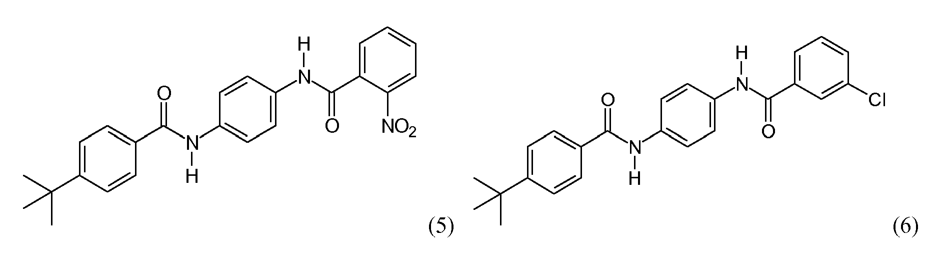

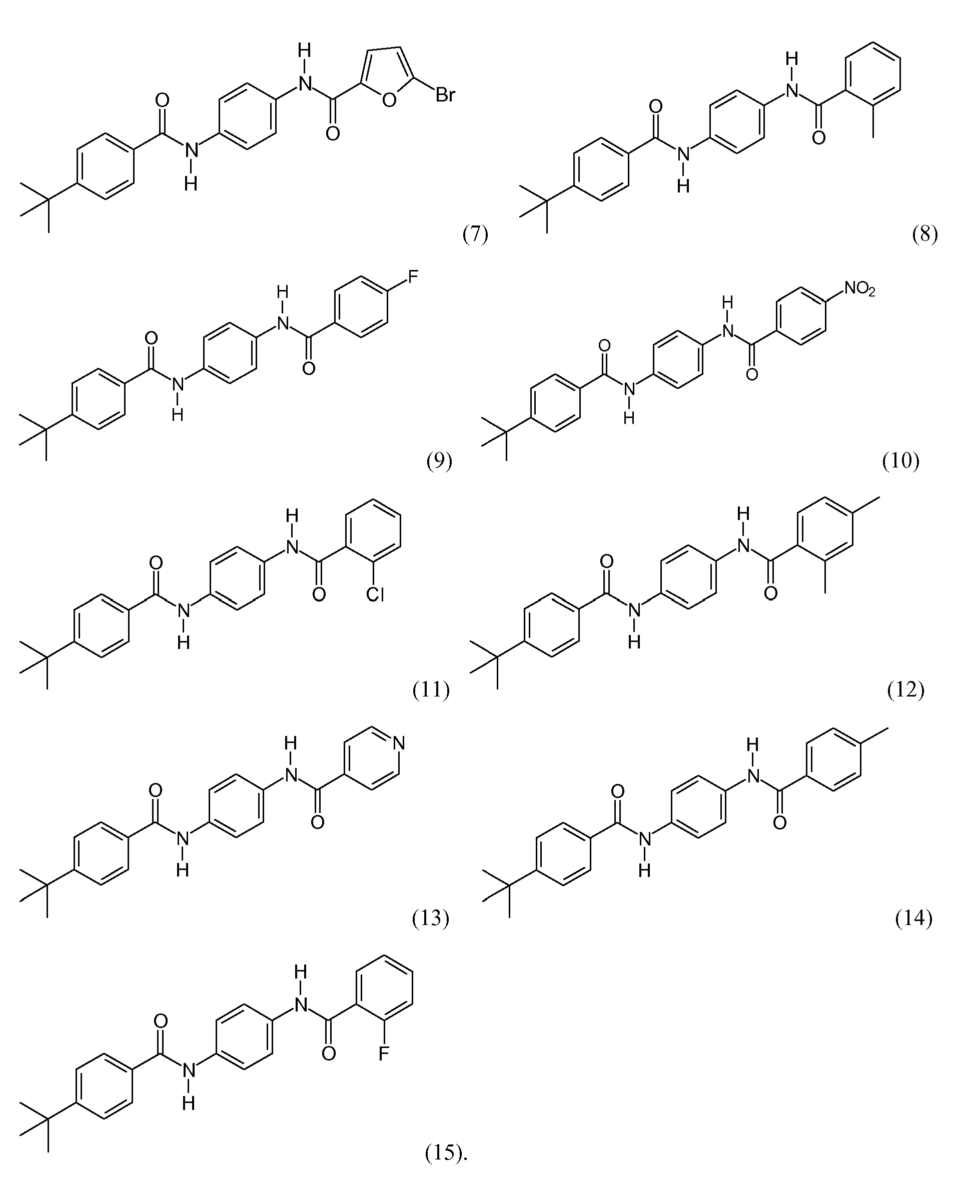

Advantageously, the phenyl diamides according to the general formulas (5) to (15) can exhibit a good inhibition of the guanine nucleotide exchange factors of the family of cytohesins and a good inhibition of glioblastoma cell proliferation.

The phenyl diamides according to the general formulas (5), (6), and (8) to (12), (14) and (15) showed to be potent inhibitors of glioblastoma cell proliferation, showing a proliferation inhibition of > 30 % or even > 50 % compared to untreated glioblastoma cells. The phenyl diamides according to the general formulas (7) and (13) exhibited a selectivity for the mutation EGFR variant III (EGFRvIII).

Another aspect of the present invention relates to the phenyl diamides according to the general formula (1) as indicated below and/or racemates, enantiomers, diastereomers, solvates, hydrates, and pharmaceutically acceptable salts and/or esters thereof: 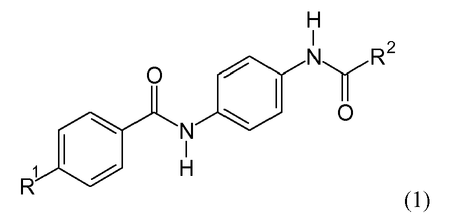 wherein:
- R¹: is selected from the group comprising hydrogen, linear or branched C₁-C₁₀-alkyl and/or linear or branched C₂-C₁₀-alkenyl;
- R²: is selected from the group comprising structural elements (2), (3) and/or (4) as given as follows: 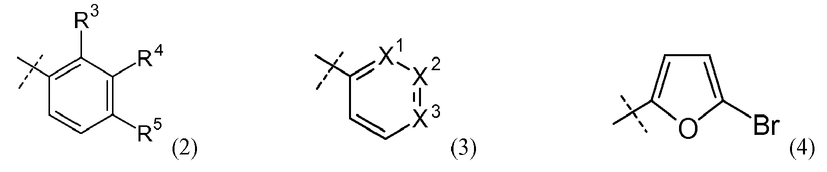 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH,
for use in the treatment of tumor diseases such as lung or bronchial cancer, colon, gastrointestinal, pancreatic or rectal cancer, prostate cancer, lymphatic cancer or leukemia, gliomas, bladder or kidney cancer, breast and/or ovarian cancer, preferably gliomas.

Surprisingly it was found that the phenyl diamides according to the general formula (1) can be effective against cancer cells. Especially, the phenyl diamides were found to effectively inhibit the growth of glioblastoma cells.

One particular advantage of the phenyl diamides according to the invention is that the phenyl diamides especially are usable for the treatment of gliomas.

Preferably, the substituent R¹ is a straight-chain or branched C₁-C₅-alkyl group selected from the group comprising methyl, ethyl, isopropyl and/or tert.-butyl, preferably a branched C₁-C₅-alkyl group selected from the group comprising isopropyl and/or tert.-butyl. Most preferably, the substituent R¹ is tert.-butyl.

Preferably, a substituent R² of the phenyl diamides is the structural element (2). Further preferred, the phenyl diamides comprise a substituent R² that is the structural element (2), wherein R³ is selected from the group comprising Cl, F, linear or branched C₁-C₄-alkyl and/or NO₂ and R⁴ and R⁵ are hydrogen. Further preferred, the phenyl diamides comprise a substituent R² that is the structural element (2), wherein R³ and R⁵ are hydrogen, and R⁴ is selected from the group comprising Cl, F, linear or branched C₁-C₄-alkyl and/or NO₂, preferably Cl. Further preferred, the phenyl diamides comprise a substituent R² that is the structural element (2), wherein R³ and R⁴ are hydrogen, and R⁵ is selected from the group comprising Cl, F, linear or branched C₁-C₄-alkyl and/or NO₂. Further preferred, the phenyl diamides comprise a substituent R² that is the structural element (2), wherein R⁴ is hydrogen, and R³ and R⁵ are selected from the group comprising Cl, F, linear or branched C₁-C₄-alkyl and/or NO₂, preferably methyl.

In other preferred embodiments, the phenyl diamides according to the general formula (1) comprise a substituent R² that is the structural element (3). In a further preferred embodiment, the phenyl diamides comprise a substituent R² that is the structural element (3), wherein X¹ and X² are CH and X³ is N. Alternatively, X¹ and X³ can be CH and X² N, or X² and X³ can be CH and X¹ N. In further embodiment, X¹ and X³, X¹ and X², or X² and X³ can be N. In a further preferred embodiment, the phenyl diamides comprise a substituent R² that is the structural element (4).

In more preferred embodiments, the phenyl diamides are selected from the group comprising the compounds (5) to (15) as indicated above and/or racemates, enantiomers, diastereomers, solvates, hydrates, and pharmaceutically acceptable salts and/or esters thereof.

Another aspect of the present invention relates to a pharmaceutical preparation comprising as an active ingredient a phenyl diamide according to the general formula (1) as indicated below and/or racemates, enantiomers, diastereomers, solvates, hydrates, and pharmaceutically acceptable salts and/or esters thereof: 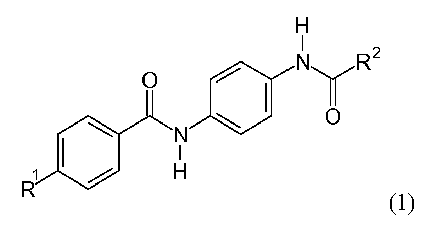 wherein:
- R¹: is selected from the group comprising hydrogen, linear or branched C₁-C₁₀-alkyl and/or linear or branched C₂-C₁₀-alkenyl;
- R²: is selected from the group comprising structural elements (2), (3) and/or (4) as given as follows: 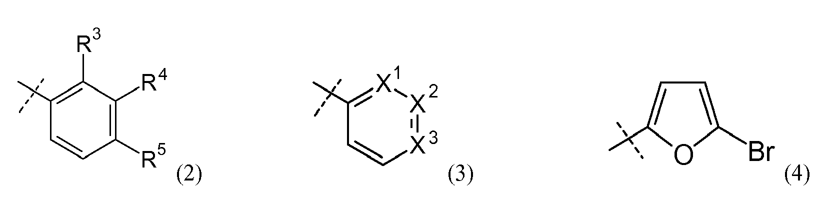 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH.

Preferably, the substituent R¹ is a straight-chain or branched C₁-C₅-alkyl group selected from the group comprising methyl, ethyl, isopropyl and/or tert.-butyl, preferably a branched C₁-C₅-alkyl group selected from the group comprising isopropyl and/or tert.-butyl. Most preferably, the substituent R¹ is tert.-butyl.

Preferably, a substituent R² of the phenyl diamides is the structural element (2). Further preferred, the phenyl diamides comprise a substituent R² that is the structural element (2), wherein R³ is selected from the group comprising Cl, F, linear or branched C₁-C₄-alkyl and/or NO₂ and R⁴ and R⁵ are hydrogen. Further preferred, the phenyl diamides comprise a substituent R² that is the structural element (2), wherein R³ and R⁵ are hydrogen, and R⁴ is selected from the group comprising Cl, F, linear or branched C₁-C₄-alkyl and/or NO₂, preferably Cl. Further preferred, the phenyl diamides comprise a substituent R² that is the structural element (2), wherein R³ and R⁴ are hydrogen, and R⁵ is selected from the group comprising Cl, F, linear or branched C₁-C₄-alkyl and/or NO₂. Further preferred, the phenyl diamides comprise a substituent R² that is the structural element (2), wherein R⁴ is hydrogen, and R³ and R⁵ are selected from the group comprising Cl, F, linear or branched C₁-C₄-alkyl and/or NO₂, preferably methyl.

In other preferred embodiments, the phenyl diamides according to the general formula (1) comprise a substituent R² that is the structural element (3). In a further preferred embodiment, the phenyl diamides comprise a substituent R² that is the structural element (3), wherein X¹ and X² are CH and X³ is N. Alternatively, X¹ and X³ can be CH and X² N, or X² and X³ can be CH and X¹ N. In further embodiment, X¹ and X³, X¹ and X², or X² and X³ can be N. In a further preferred embodiment, the phenyl diamides comprise a substituent R² that is the structural element (4).

In more preferred embodiments, the phenyl diamides are selected from the group comprising the compounds (5) to (15) as indicated above and/or racemates, enantiomers, diastereomers, solvates, hydrates, and pharmaceutically acceptable salts and/or esters thereof.

In preferred embodiments, the pharmaceutical preparation is usable in the treatment of tumor diseases such as lung or bronchial cancer, colon, gastrointestinal, pancreatic or rectal cancer, prostate cancer, lymphatic cancer or leukemia, gliomas, bladder or kidney cancer, breast and/or ovarian cancer, preferably gliomas.

The present invention also relates to the use of phenyl diamides according to the general formula (1) as indicated below and/or racemates, enantiomers, diastereomers, solvates, hydrates, and pharmaceutically acceptable salts and/or esters thereof: 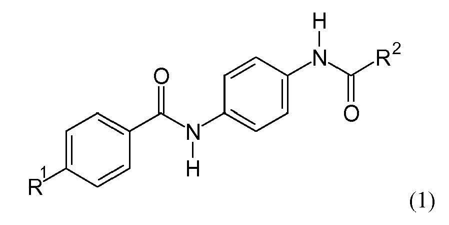 wherein:
- R¹: is selected from the group comprising hydrogen, linear or branched C₁-C₁₀-alkyl and/or linear or branched C₂-C₁₀-alkenyl;
- R²: is selected from the group comprising structural elements (2), (3) and/or (4) as given as follows: 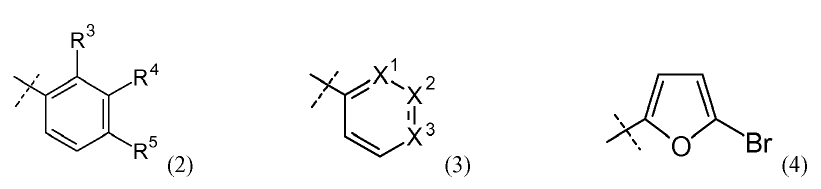 wherein:
R³, R⁴, R⁵, are selected, the same or each independently of the others, from the group comprising hydrogen, halogen, preferably selected from the group comprising Cl, Br, F, linear or branched C₅-C₅-alkyl and/or NO₂;
X¹, X², X³, are selected, the same or each independently of the others, from the group comprising N and/or CH,
for the manufacture of a medicament.

Preferably, the substituent R¹ is a straight-chain or branched C₁-C₅-alkyl group selected from the group comprising methyl, ethyl, isopropyl and/or tert.-butyl, preferably a branched C₁-C₅-alkyl group selected from the group comprising isopropyl and/or tert.-butyl. Most preferably, the substituent R¹ is tert.-butyl.

Preferably, a substituent R² of the phenyl diamides is the structural element (2). Further preferred, the phenyl diamides comprise a substituent R² that is the structural element (2), wherein R³ is selected from the group comprising Cl, F, linear or branched C₁-C₄-alkyl and/or NO₂ and R⁴ and R⁵ are hydrogen. Further preferred, the phenyl diamides comprise a substituent R² that is the structural element (2), wherein R³ and R⁵ are hydrogen, and R⁴ is selected from the group comprising Cl, F, linear or branched C₁-C₄-alkyl and/or NO₂, preferably Cl. Further preferred, the phenyl diamides comprise a substituent R² that is the structural element (2), wherein R³ and R⁴ are hydrogen, and R⁵ is selected from the group comprising Cl, F, linear or branched C₁-C₄-alkyl and/or NO₂. Further preferred, the phenyl diamides comprise a substituent R² that is the structural element (2), wherein R⁴ is hydrogen, and R³ and R⁵ are selected from the group comprising Cl, F, linear or branched C₁-C₄-alkyl and/or NO₂, preferably methyl.

In other preferred embodiments, the phenyl diamides according to the general formula (1) comprise a substituent R² that is the structural element (3). In a further preferred embodiment, the phenyl diamides comprise a substituent R² that is the structural element (3), wherein X¹ and X² are CH and X³ is N. Alternatively, X¹ and X³ can be CH and X² N, or X² and X³ can be CH and X¹ N. In further embodiment, X¹ and X³, X and X², or X² and X³ can be N. In a further preferred embodiment, the phenyl diamides comprise a substituent R² that is the structural element (4).

In more preferred embodiments, the phenyl diamides are selected from the group comprising the compounds (5) to (15) as indicated above and/or racemates, enantiomers, diastereomers, solvates, hydrates, and pharmaceutically acceptable salts and/or esters thereof.

A further aspect of the present invention relates to the use of phenyl diamides according to the general formula (1) as indicated below and/or racemates, enantiomers, diastereomers, solvates, hydrates, and pharmaceutically acceptable salts and/or esters thereof: 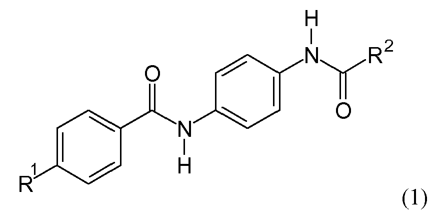 wherein:
- R¹: is selected from the group comprising hydrogen, linear or branched C₁-C₁₀-alkyl and/or linear or branched C₂-C₁₀-alkenyl;
- R²: is selected from the group comprising structural elements (2), (3) and/or (4) as given as follows: 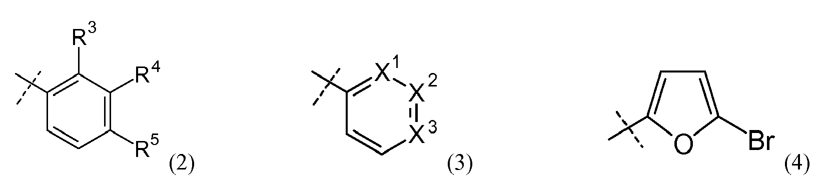 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH,
for the manufacture of a medicament for the treatment of tumor diseases such as lung or bronchial cancer, colon, gastrointestinal, pancreatic or rectal cancer, prostate cancer, lymphatic cancer or leukemia, gliomas, bladder or kidney cancer, breast and/or ovarian cancer, preferably gliomas.

It is a particular advantage of the phenyl diamides according to the invention is that the phenyl diamides especially are usable for the manufacture of a medicament for the treatment of gliomas.

Preferably, the substituent R¹ is a straight-chain or branched C₁-C₅-alkyl group selected from the group comprising methyl, ethyl, isopropyl and/or tert.-butyl, preferably a branched C₁-C₅-alkyl group selected from the group comprising isopropyl and/or tert.-butyl. Most preferably, the substituent R¹ is tert.-butyl.

Preferably, a substituent R² of the phenyl diamides is the structural element (2). Further preferred, the phenyl diamides comprise a substituent R² that is the structural element (2), wherein R³ is selected from the group comprising Cl, F, linear or branched C₁-C₄-alkyl and/or NO₂ and R⁴ and R⁵ are hydrogen. Further preferred, the phenyl diamides comprise a substituent R² that is the structural element (2), wherein R³ and R⁵ are hydrogen, and R⁴ is selected from the group comprising Cl, F, linear or branched C₁-C₄-alkyl and/or NO₂, preferably Cl. Further preferred, the phenyl diamides comprise a substituent R² that is the structural element (2), wherein R³ and R⁴ are hydrogen, and R⁵ is selected from the group comprising Cl, F, linear or branched C₁-C₄-alkyl and/or NO₂. Further preferred, the phenyl diamides comprise a substituent R² that is the structural element (2), wherein R⁴ is hydrogen, and R³ and R⁵ are selected from the group comprising Cl, F, linear or branched C₁-C₄-alkyl and/or NO₂, preferably methyl.

In other preferred embodiments, the phenyl diamides according to the general formula (1) comprise a substituent R² that is the structural element (3). In a further preferred embodiment, the phenyl diamides comprise a substituent R² that is the structural element (3), wherein X¹ and X² are CH and X³ is N. Alternatively, X¹ and X³ can be CH and X² N, or X² and X³ can be CH and X¹ N. In further embodiment, X¹ and X³, X¹ and X², or X² and X³ can be N. In a further preferred embodiment, the phenyl diamides comprise a substituent R² that is the structural element (4).

In more preferred embodiments, the phenyl diamides are selected from the group comprising the compounds (5) to (15) as indicated above and/or racemates, enantiomers, diastereomers, solvates, hydrates, and pharmaceutically acceptable salts and/or esters thereof.

A further aspect of the present invention relates to phenyl diamides according to the formulas (16), (17) and (18) as indicated below and/or racemates, enantiomers, diastereomers, solvates, hydrates, and pharmaceutically acceptable salts and/or esters thereof: 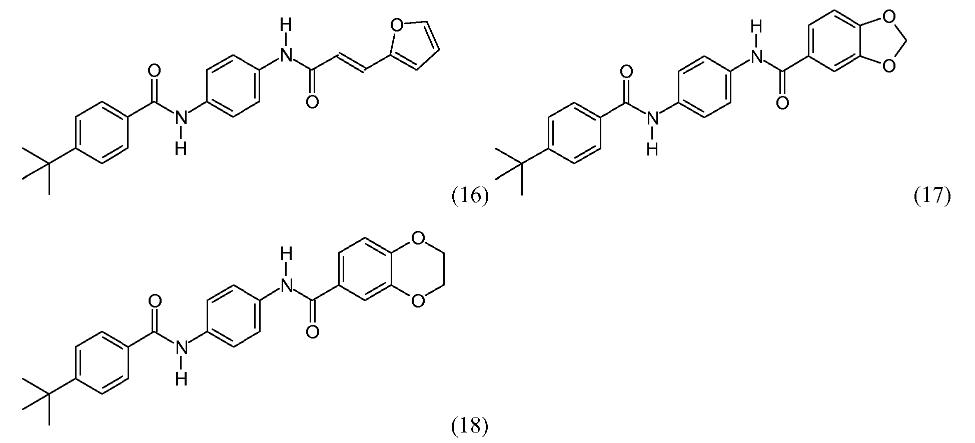 for use in the treatment of gliomas.

The phenyl diamides according to the general formula (1), especially according to the general formulas (5) to (15) and (16) to (18) can be synthesized according to standard chemical methods. The compound according to formula (16) is denoted according to the IUPAC (International Union of Pure and Applied Chemistry) nomenclature 4-tert-butyl-N-[4-[[(E)-3-furan-2-ylprop-2-enoyl]amino]phenyl]benzamide, the compound according to formula (5) is denoted N-{4-[(4-tert-butylbenzoyl)amino]phenyl}-2-nitrobenzamide, and the compound according to formula (6) is denoted N- {4-[(4-tert-butylbenzoyl)amino]phenyl}-3-chlorobenzamide, respectively.

Surprisingly it was found that the phenyl diamides according to the formulas (16), (17) and (18) exhibit a strong inhibition of the guanine nucleotide exchange factors of the family of cytohesins and are potent inhibitors of glioblastoma cell proliferation.

Especially, the phenyl diamide according to the formulas (16) exhibited a strong inhibition of the guanine nucleotide exchange factors of the family of cytohesins and are potent inhibitors of glioblastoma cell proliferation. Advantageously, the phenyl diamide according to the formula (17) exhibited a selectivity for the mutation EGFR variant III (EGFRvIII), which can be especially usable in a pharmacological treatment of gliomas. Further advantageously, the phenyl diamide according to the formula (18) showed to be a potent inhibitor of glioblastoma cell proliferation, having an IC₅₀ for the proliferation inhibition of < 2 µM.

A further aspect of the present invention relates to phenyl diamides according to the formulas (16), (17) and (18) as indicated below and/or racemates, enantiomers, diastereomers, solvates, hydrates, and pharmaceutically acceptable salts and/or esters thereof: 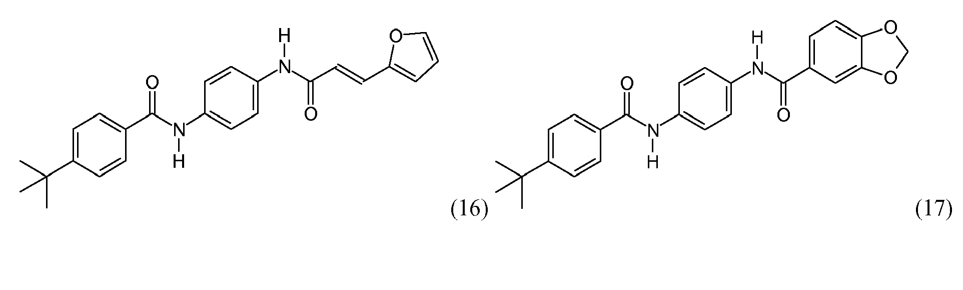 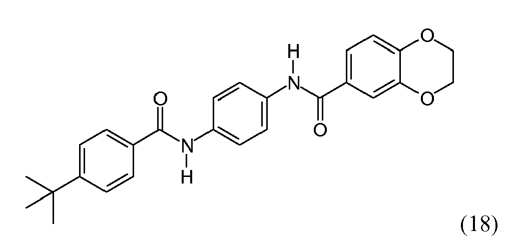 for the manufacture of a medicament for the treatment of gliomas.

Advantageously, the phenyl diamides according to the invention can have only a slight or negligible toxicity when administered. This enables a long-term use, for example.

The phenyl diamides and/or the pharmaceutical preparation can thus be administered with usual methods such as, for example, orally, dermally and/or intravenously. Oral or parenteral administration is preferred, with oral administration being especially preferred. In preferred embodiments, the pharmaceutical preparation is formulated for oral or intravenous administration.

The Examples which follow serve to illustrate the invention in more detail but do not constitute a limitation thereof.

The figures show:
- Figure 1: the relative exchange rate from guanidine-diphosphate (GDP) to guanidine-triphosphate (GTP) in the presence of the phenyl diamides according to formulas (5) to (18);
- Figure 2: the inhibition of glioblastoma cell proliferation after treatment with 0,25 µM of the phenyl diamides according to formulas (5) to (18) in the wild type glioblastoma cell line U87 wt;
- Figure 3: the inhibition of glioblastoma cell proliferation after treatment with 0,25 µM of the phenyl diamides according to formulas (5) to (18) in the wild type glioblastoma cell line Gli36 wt;
- Figure 4: the inhibition of glioblastoma cell proliferation after treatment with the phenyl diamides according to formulas (10), (13) and (16) in the wild type glioblastoma cell line U87 wt and the EGFRvIII-containing cell line U87 EGFRvIII.

The compounds according to formulas (5) to (18) are commercially accessible. The compounds according to formulas (5) and (6) were purchased from ChemBridge Corporation, San Diego, USA. The compounds according to formulas (7) and (16) were purchased from Asinex Ltd., Moscow, Russia. The compounds according to formulas (8), (9), (10), (11), (13), (14) and (15) were purchased from Aronis, Moscow, Russia. The compounds according to formulas (12), (17) and (18) were purchased from Vitas-M Laboratory Ltd., Moscow, Russia.

### Example 1

### Determination of the inhibition of the guanine nucleotide exchange activity

The inhibition of the exchange activity of the sec7-domain of cytohesin-2 (ARNO) of the phenyl diamides according to formulas (5) to (18) was determined by a guanine nucleotide exchange assay.

The sec7-domain of cytohesin-2 (ARNO) and [Δ17]ARF1, a mutant of the ADP-ribosylation factor (ARF) protein, were subcloned into pET15 vectors (Novagen) as described in Hafner, M. et al. Nature 444, 941-944 (2006). N-terminal truncated [Δ17]ARF1 (amino acids 18-181), lacking the first 17 amino acids and ARNO-Sec7 (amino acids 50-255 of ARNO) were expressed in *Escherichia coli* and purified by Ni-NTA chromatography (Ni-NTA agarose, Qiagen).

The exchange from guanidine-diphosphate (GDP) to guanidine-triphosphate (GTP) induces a conformation change of the ribosylation factor, as a result of which the fluorescence of a tryptophan radical of the protein is increased. GDP/GTP exchange was measured on [Δ17]ART1 by tryptophan fluorescence because a large increase in intrinsic fluorescence of ARF occurs upon exchange of GDP for GTP. All measurements were performed in PBS (137 mM NaCl, 2.7 mM KCl, 10.2 mM Na₂HPO₄, 1.8 mM KH₂PO₄) pH 7.4, 3 mM MgCl₂ at 37°C.

[Δ17]ARF1 (1 µM) in PBS without Macl₂ was preincubated with 80 µM GDP in the presence of 2 mM ethylenediaminetetraacetic acid (EDTA) for 15 minutes. The bound GDP was stabilized by addition of MgCl₂ at a final concentration of 3 mM and incubation for 5 minutes. For each exchange reaction 250 nM [Δ17]ARF1 was mixed with 10 nM ARNO-Sec7 (total volume 200 µl) in the absence or presence of the phenyl diamides according to formulas (5) to (18). The reaction was started by injection of 50 µM GTP.

The tryptophan fluorescence was measured at excitation and emission wavelength of 280 nm and 340 nm respectively. All fluorescent measurements were performed with a Varioskan microplate reader (Thermo Scientific), in 96-well plates. For analysis all data were fitted by linear regression.

The relative exchange rate was determined by comparing the initial linear rise of the fluorescence signal (exchange rate) in the presence of 2.5 µM of the phenyl diamides according to formulas (5) to (18) with the exchange rate in the presence of the solvent Dimethyl sulfoxide (DMSO), 0.4% end concentration, only. A further control was performed with the control compound according to formula (XH1009) as indicated below:

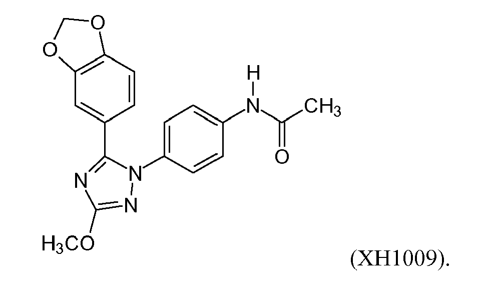

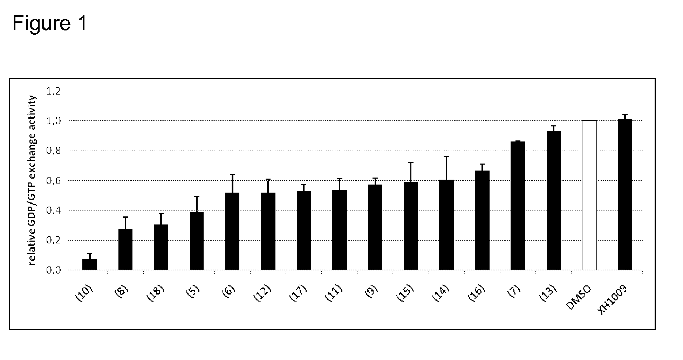

As can be seen in Figure 1, all phenyl diamides exhibited a decline in the relative GDP/GTP exchange activity and hence an inhibition of the sec7-domain of the isolated cytohesin-2 compared to the controls.

Further, the IC₅₀ value of the inhibition of the sec7-domain of the isolated cytohesin-2 was determined. The IC₅₀ value was determined by plotting the initial linear rise of the fluorescence signal against the added concentrations of the amide. The IC₅₀ value corresponds to the concentration of the phenyl diamides at which the activity of the protein is reduced to half. The lower the IC₅₀ value, the more strongly the phenyl diamide inhibits the sec7-domain.

The IC50 values measured for the isolated Sec-7 domain of cytohesin-2 of the phenyl diamides according to formulas (5), (6), (7), (8), (9), (10), (11), (12), (13), ),(14), (15), (16), (17), and (18) have been determined to 1.18 µM +/- 0.17 µM, 0.79 µM +/- 0,15 µM, 4.57 µM +/-0.33 µM, 1.19 µM+/-0.15 µM, 2.1 µM +/- 0.15 µM, 0.44 µM +/-0.06 µM, 2.58 µM +/- 0.18 µM, 1.86 µM +/- 0.3 µM, 7.27 µM +/- 0.56 µM, 1.76 µM +/- 0.22 µM, 1.77µM +/- 0.23 µM, 3.75 µM +/- 0.37 µM, 2.69 µM +/-0.14 µM, and 1.34 µM +/-0.16 µM, respectively. Each individual experiment was performed at least 8 times.

This experiment shows, that the phenyl diamides are very potent inhibitors of the cytohesin guanine nucleotide exchange activity.

### Example 2

### Glioblastoma Proliferation assay

The effect of the phenyl diamides according to formulas (5) to (18) on the proliferation of Glioblastoma cells was tested in two pairs of established human glioblastoma cell lines, Gli36 and U87.

Human Gli36 wt, U87 wt and U87 EGFRvIII cells were a gift from Prof. Dr. Andreas H. Jacobs, European Institute for Molecular Imaging, Munster, Germany. The cells were maintained in DMEM (PAA) supplemented with 10% fetal calf serum (FCS, Lonza), 1% Penicillin/Streptomycin (PAA) and 110 µg/ml Sodiumpyruvat (Sigma-Aldrich) and were grown at 37°C, 5% CO₂.

3000 cells per 96 well were seeded into a clear, flat bottom 96 well plate (TPP). After 24 hours the cells were washed twice in 1 x DPBS (PAA) and treated with 250 nM of the phenyl diamides according to formulas (5) to (18) in DMEM (PAA) supplemented with 1% FCS (Lonza), 1% Penicillin/Streptomycin (PAA) and 110µg/ml Sodiumpyruvat (Sigma-Aldrich). Controls were treated with Dimethyl sulfoxide (DMSO) as solvent in an end concentration of 0.4 % (v/v). A further control was performed with the control compound according to formula (XH1009). Medium was changed daily.

After 72 hours cell proliferation was quantified by using a commercially available 3-(4,5-dimethylthiazol-2-yl)-2,5diphenyltetrazolium bromide (MTT) assay (Cell Titer 96® Non-Radioactive Cell Proliferation Assay, Promega) as described in the manufacture's protocol. Briefly, 15 µl of Dye solution was added directly into the 100 µl media of the cells. After 4 hours incubation at 37°C, 5 % CO₂, 100 µl solubilization solution was added to solubilize the formazan product and the absorbance at 570 nm was measured in a Varioscan microplate reader (Thermo Scientific). All assays were performed at least in triplicates. For calculation of the relative proliferation rate/cell number the mean absorbance in the solvent DMSO only treated cells was set as 1. Results are given as the mean +/standard error.

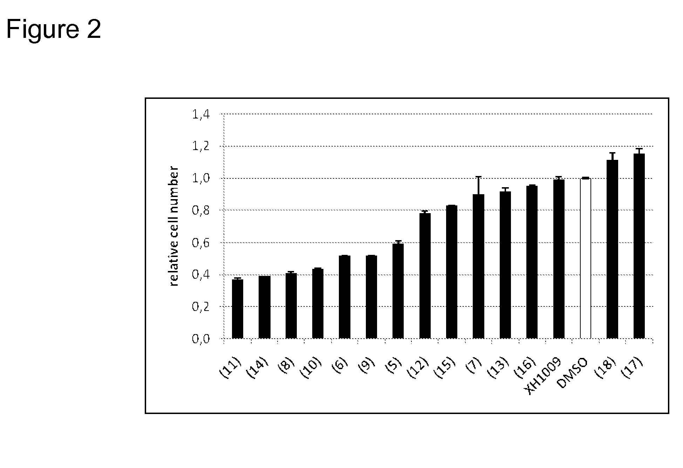

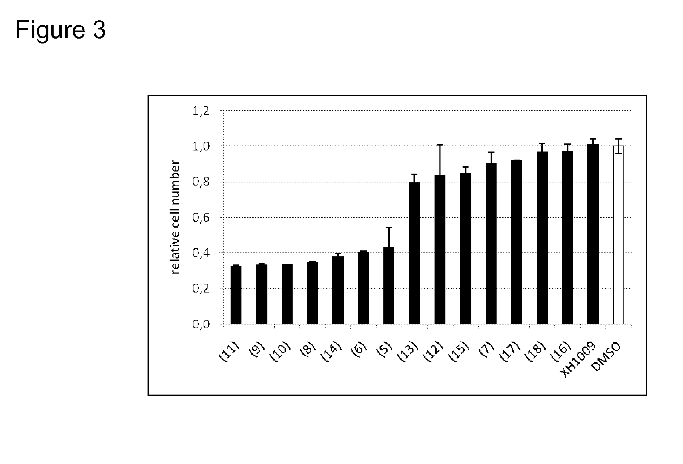

The figures 2 and 3 show the relative cell numbers in the wild type glioblastoma cell line U87 wt (Figure 2) and Gli36 wt (Figure 3) normalized to the DSMO treated control after 72 h incubation with the respective phenyl diamides according to formulas (5) to (18), the DSMO treated control compound determined with a MTT proliferation essay. Data were normalized with the exchange in the solvent (DMSO) only control set as 1 and represent the mean values of duplicates, error bars correspond to standard error of the mean (SEM).

It can be seen that the phenyl diamides according to formulas (8), (10), (11), and (14) showed an inhibitory effect of more than 50% as compared to control in both cell lines Gli36 wt and U87 wt. The phenyl diamides according to formulas (5), (6), and (9) decreased the cell proliferation in both cell lines between 30% and 50%.

A comparison of the two parental cell lines U87 wt and Gli36 wt revealed a significant correlation (p<0,0001, r2=0,82) between the inhibitory effects of the phenyl diamides on the proliferation of both cell lines.

This experiment shows that the phenyl diamides according to formulas (5) to (18) exhibit a potent inhibition of the cell proliferation in glioblastoma cells.

Further, the inhibitory activity of the compounds in the exchange assay with their effect on proliferation of the parental cell lines U87wt and Gli36wt was correlated. Scatter blots revealed a significant positive correlation (p<0,0001, r2=0,35 U87, r2=0,45 Gli36, data normalized, the phenyl diamide according to formulas (16) set to 1) between the inhibitory effect of the compounds at a concentration of 2.5 µM on the exchange activity of the cytohesins and the inhibition of cell proliferation at a concentration of 250 nM in both parental cell lines.

Such a correlation between the inhibitory activities of the compounds in the exchange assay and on cell proliferation, provides evidence that a decreased proliferation is a consequence of a decreased cytohesin activity.

### Example 3

### Determination of the selectivity of the inhibition

The selectivity of the inhibition of the EGFRvIII dependent cell proliferation was determined by comparing the effect of the phenyl diamides according to formulas (5) to (18) on the parental and the respective isogenenic EGFRvIII transfected cell line U87 EGFRvIII, which differs only in the expression of the stably transfected EGFRvIII.

U87 wt and U87 EGFRvIII cells were a gift from Prof. Dr. Andreas H. Jacobs, European Institute for Molecular Imaging, Münster, Germany. The cells were maintained in DMEM (PAA) supplemented with 1% FCS (Lonza), 1% Penicillin/Streptomycin (PAA) and 110 µg/ml Sodiumpyruvat (Sigma-Aldrich) and were grown at 37°C, 5% CO₂.

300 cells per 96 well were seeded into a clear, flat bottom 96 well plate (TPP). After 24 hours the cells were washed twice in 1 x DPBS (PAA) and treated with concentrations ranging from 2.5 nM to 2 µM of the phenyl diamides according to formulas (5) to (18) or 5 µM of the control compound (XH1009) in DMEM (PAA) supplemented with 1% FCS (Lonza), 1% Penicillin/Streptomycin (PAA) and 110µg/ml Sodiumpyruvat (Sigma-Aldrich). Controls were treated with Dimethyl sulfoxide (DMSO) as solvent in an end concentration of 0.4 % (v/v). Medium was changed daily.

After 72 hours cell proliferation was quantified by using a commercially available 3-(4,5-dimethylthiazol-2-yl)-2,5diphenyltetrazolium bromide (MTT) assay (Cell Titer 96® Non-Radioactive Cell Proliferation Assay, Promega) as described in the manufacture's protocol. Briefly, 15 µl of Dye solution was added directly into the 100 µl media of the cells. After 4 hours incubation at 37°C, 5% CO₂, 100 µl solubilization solution was added to solubilize the formazan product and the absorbance at 570 nm was measured in a Varioscan microplate reader (Thermo Scientific). All assays were performed at least in triplicates. For calculation of the relative proliferation rate/cell number the mean absorbance in the solvent DMSO only treated cells was set as 1. Results are given as the mean +/standard error.

Further, the IC₅₀ values of the inhibition of the proliferation was determined. The IC₅₀ value was determined by plotting the relative cell number against the added concentrations of the amide. The IC₅₀ value corresponds to the concentration of the phenyl diamides at which the proliferation is reduced to half. The lower the IC₅₀ value, the more strongly the phenyl diamide inhibits proliferation.

The IC₅₀ values measured in the cell line U87 wt for the phenyl diamides according to formulas (5), (6), (7), (8), (9), (10), (11), (12), (13), (16), (17), and (18) have been determined to 168 nM, 243 nM, 1.37 µM, 118 nM, 89 nM, 28 nM, 106 nM, 367 nM, 2.09 µM, 958 nM, 922 nM, and 297 nM, respectively. Each individual experiment was performed at least 3 times.

The IC50 values measured in the cell line U87 EGFRvIII for the phenyl diamides according to formulas (5), (6), (7), (8), (9), (10), (11), (12), (13), (16), (17), and (18) have been determined to 46 nM +/- 1.2 nM, 67 nM +/- 1.2 nM, 198 nM +/- 1.3 nM, 148 nM +/- 1.1 nM, 372 nM +/- 1.5 nM, 83 nM +/- 1.2 nM, 126 nM +/- 1.2 nM, 84 nM +/- 1.1 nM, 186 nM +/- 1.2 nM, 435 nM +/- 1.3 nM, 224 nM +/- 1.3 nM, and 105 nM +/- 1.1 nM, respectively. Each individual experiment was performed at least 3 times.

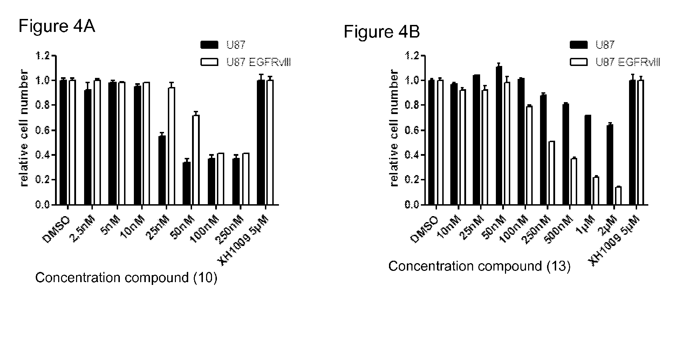

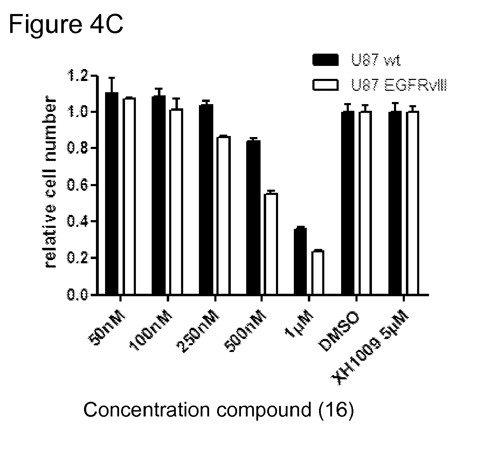

Figure 4 shows the inhibition of glioblastoma cell proliferation after treatment with the phenyl diamides according to formulas (10) (Figure 4A), (13) (Figure 4B) and (16) (Figure 4C) in the wild type glioblastoma cell line U87 wt and the EGFRvIII-containing cell line U87 EGFRvIII compared to the controls treated with DMSO only or the compound (XH1009). The selectivity of the inhibition of the EGFRvIII dependent cell proliferation was determined by comparing the effect of the phenyl diamides according to formulas (10), (13) and (16) on the parental and the respective isogenenic EGFRvIII transfected cell line U87 EGFRvIII, which differs only in the expression of the stably transfected EGFRvIII.

As can be seen from Figure 4, the phenyl diamide according to formula (10) shows a selectively for the inhibition of the parental U87 wt cell line (Figure 4A), whereas phenyl diamide according to formula (13) selectively inhibits the EGFRvIII expressing U87 cell line (Figure 4B). The phenyl diamide according to formula (16) shows no selectively (Figure 4C).

A comparison of the inhibitory effects of the phenyl diamides on the proliferation of the parental cell line with the effect on the respective EGFRvIII-transfected cell line U87 EGFRvIII showed that the phenyl diamides according to formulas (7), (13), and (17) selectively inhibited the proliferation of the U87 EGFRvIII-containing cell line. For example diminished the phenyl diamide according to formula (13) the proliferation of the EGFRvIII-cells more than 10-fold better than that of the parental cell line, as can be seen from Figure 4B.

This shows a potent inhibition of cell proliferation in glioblastoma cell lines by the phenyl diamides according to formulas (5) to (18) and a selectivity of the inhibition for the phenyl diamides according to formulas (7), (13), and (17).

## Claims

1. Phenyl diamides according to the general formula (1) as indicated below and/or racemates, enantiomers, diastereomers, solvates, hydrates, and pharmaceutically acceptable salts and/or esters thereof: 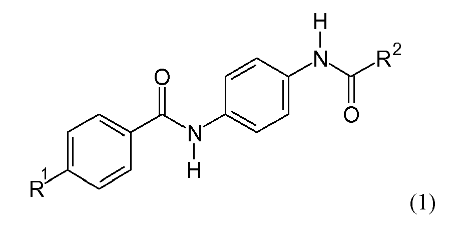 wherein:
R¹ is selected from the group comprising hydrogen, linear or branched C₁-C₁₀-alkyl and/or linear or branched C₂-C₁₀-alkenyl;
R² is selected from the group comprising structural elements (2), (3) and/or (4) as given as follows: 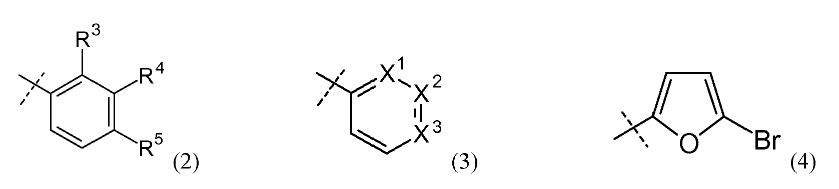 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH,
for use as a medicament.

2. The phenyl diamides according to claim 1, **characterized in that** R² is the structural element (2), wherein R³ is selected from the group comprising Cl, F, linear or branched C₁-C₄-alkyl and/or NO₂, and R⁴ and R⁵ are hydrogen.

3. The phenyl diamides according to claim 1, **characterized in that** R² is the structural element (2), wherein R³ and R⁵ are hydrogen, and R⁴ is selected from the group comprising Cl, F, linear or branched C₁-C₄-alky, and/or NO₂, preferably Cl.

4. The phenyl diamides according to claim 1, **characterized in that** R² is the structural element (2), wherein R³ and R⁴ are hydrogen, and R⁵ is selected from the group comprising Cl, F, linear or branched C₁-C₄-alkyl and/or NO₂.

5. The phenyl diamides according to claim 1, **characterized in that** R² is the structural element (2), wherein R⁴ is hydrogen, and R³ and R⁵ are selected from the group comprising Cl, F, linear or branched C₁-C₄-alkyl and/or NO₂, preferably methyl.

6. The phenyl diamides according to claim 1, **characterized in that** R² is the structural element (3), wherein X¹ and X² are CH and X³ is N.

7. The phenyl diamides according to claim 1, **characterized in that** R² is the structural element (4).

8. The phenyl diamides according to any of the preceding claims, **characterized in that** the phenyl diamides are selected from the group comprising the compounds (5) to (15) as indicated below and/or racemates, enantiomers, diastereomers, solvates, hydrates, and pharmaceutically acceptable salts and/or esters thereof:

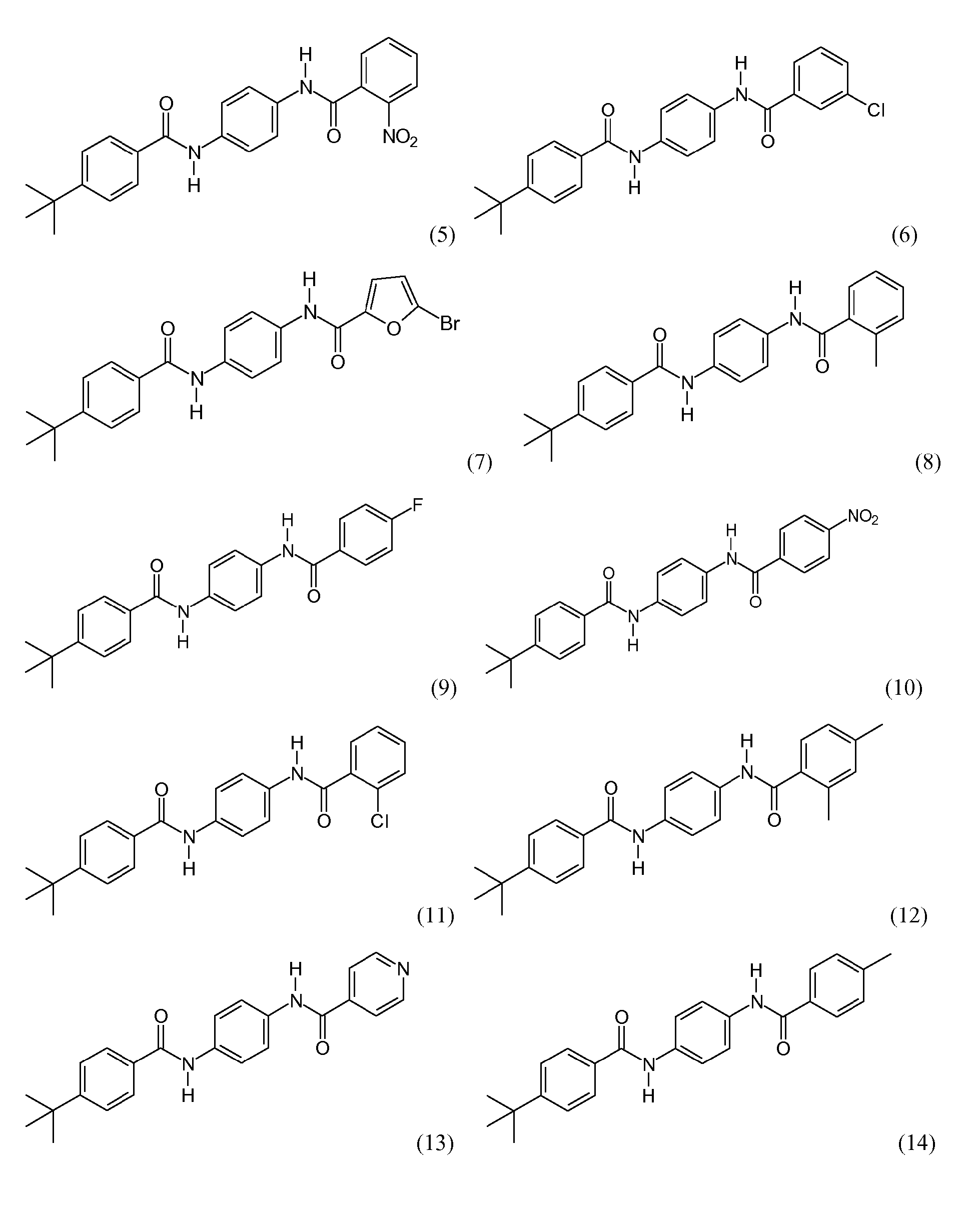

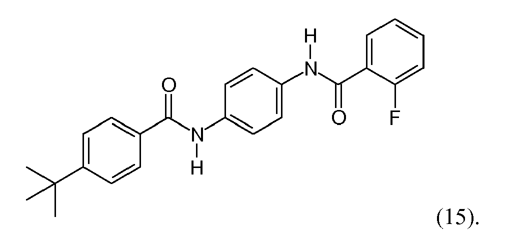

9. Phenyl diamides according to the general formula (1) as indicated below and/or racemates, enantiomers, diastereomers, solvates, hydrates, and pharmaceutically acceptable salts and/or esters thereof: 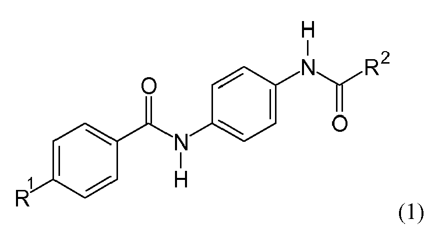 wherein:
R¹ is selected from the group comprising hydrogen, linear or branched C₁-C₁₀-alkyl and/or linear or branched C₂-C₁₀-alkenyl;
R² is selected from the group comprising structural elements (2), (3) and/or (4) as given as follows: 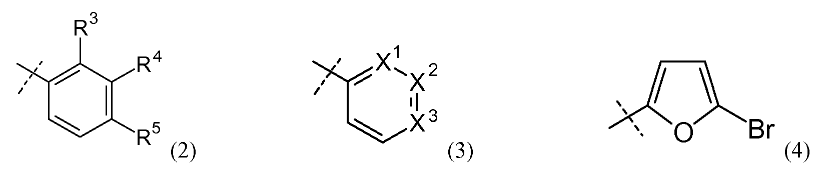 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH,
for use in the treatment of tumor diseases such as lung or bronchial cancer, colon, gastrointestinal, pancreatic or rectal cancer, prostate cancer, lymphatic cancer or leukemia, gliomas, bladder or kidney cancer, breast and/or ovarian cancer, preferably gliomas.

10. A pharmaceutical preparation comprising as an active ingredient a phenyl diamide according to the general formula (1) as indicated below and/or racemates, enantiomers, diastereomers, solvates, hydrates, and pharmaceutically acceptable salts and/or esters thereof: 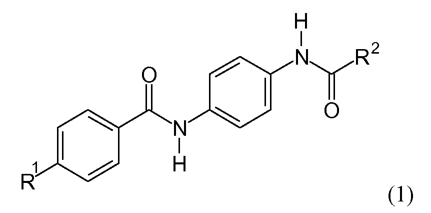 wherein:
R¹ is selected from the group comprising hydrogen, linear or branched C₁-C₁₀-alkyl and/or linear or branched C₂-C₁₀-alkenyl;
R² is selected from the group comprising structural elements (2), (3) and/or (4) as given as follows: 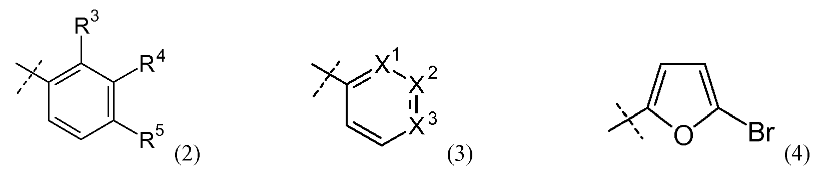 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH.

11. The pharmaceutical preparation according to claim 10 for use in the treatment of tumor diseases such as lung or bronchial cancer, colon, gastrointestinal, pancreatic or rectal cancer, prostate cancer, lymphatic cancer or leukemia, gliomas, bladder or kidney cancer, breast and/or ovarian cancer, preferably gliomas.

12. Use of phenyl diamides according to the general formula (1) as indicated below and/or racemates, enantiomers, diastereomers, solvates, hydrates, and pharmaceutically acceptable salts and/or esters thereof: 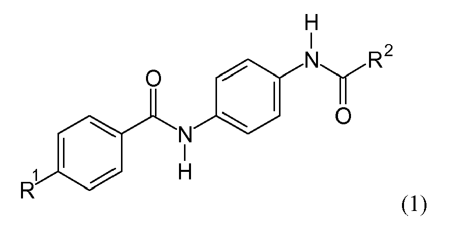 wherein:
R¹ is selected from the group comprising hydrogen, linear or branched C₁-C₁₀-alkyl and/or linear or branched C₂-C₁₀-alkenyl;
R² is selected from the group comprising structural elements (2), (3) and/or (4) as given as follows: 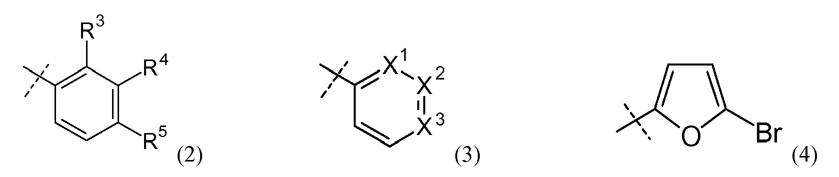 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH,
for the manufacture of a medicament.

13. The use of phenyl diamides according to the general formula (1) as indicated below and/or racemates, enantiomers, diastereomers, solvates, hydrates, and pharmaceutically acceptable salts and/or esters thereof: 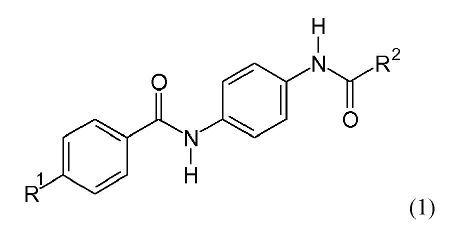 wherein:
R¹ is selected from the group comprising hydrogen, linear or branched C₁-C₁₀-alkyl and/or linear or branched C₂-C₁₀-alkenyl;
R² is selected from the group comprising structural elements (2), (3) and/or (4) as given as follows: 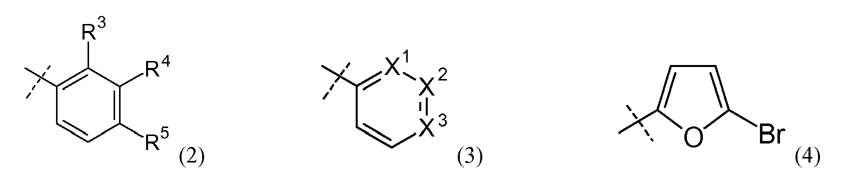 wherein:
R³, R⁴, R⁵, are selected, the same or each independently of the others, from the group comprising hydrogen, halogen, preferably selected from the group comprising Cl, Br, F, linear or branched C₁-C₅-alkyl and/or NO₂;
X¹, X², X³, are selected, the same or each independently of the others, from the group comprising N and/or CH,
for the manufacture of a medicament for the treatment of tumor diseases such as lung or bronchial cancer, colon, gastrointestinal, pancreatic or rectal cancer, prostate cancer, lymphatic cancer or leukemia, gliomas, bladder or kidney cancer, breast and/or ovarian cancer, preferably gliomas.

14. Phenyl diamides according to the formulas (16), (17) and (18) as indicated below and/or racemates, enantiomers, diastereomers, solvates, hydrates, and pharmaceutically acceptable salts and/or esters thereof: 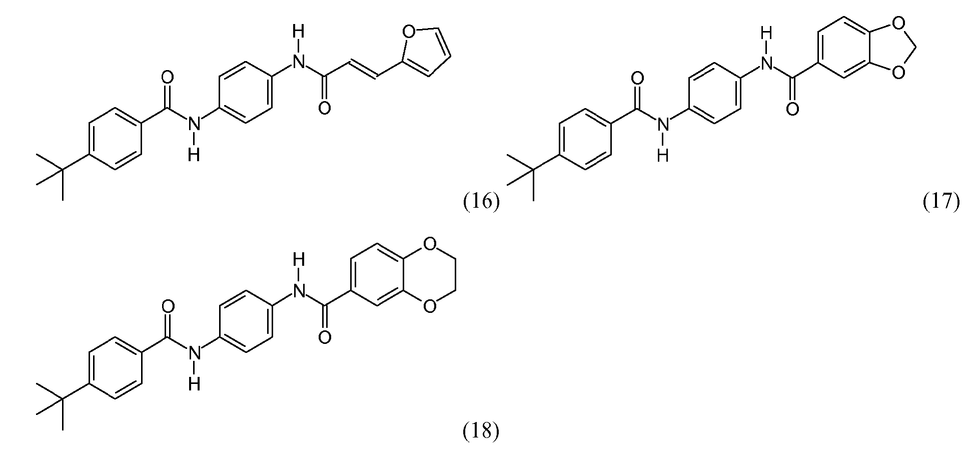 for use in the treatment of gliomas.

15. Phenyl diamides according to the formulas (16), (17) and (18) as indicated below and/or racemates, enantiomers, diastereomers, solvates, hydrates, and pharmaceutically acceptable salts and/or esters thereof: 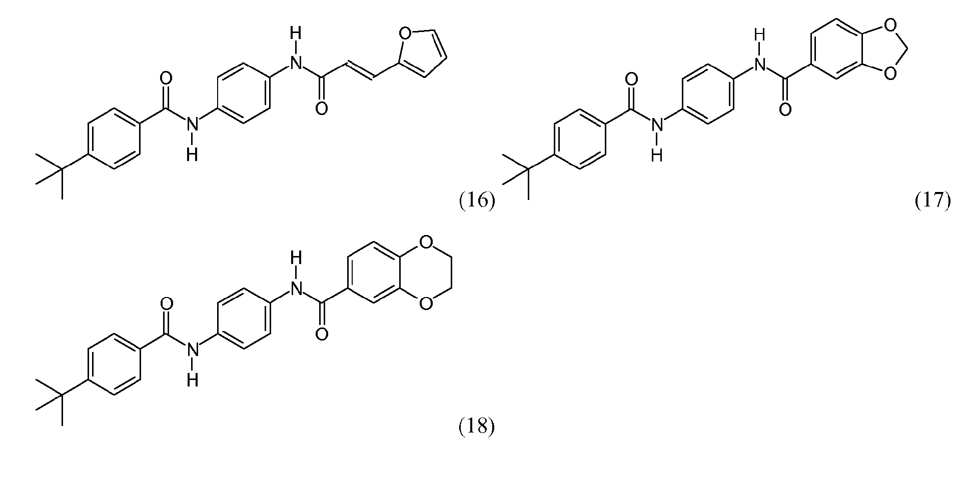 for the manufacture of a medicament for the treatment of gliomas.
